# EUROPÄISCHE PATENTSCHRIFT

(11) **EP 1 363 872 B1**
(45) Veröffentlichungstag und Bekanntmachung des Hinweises auf die Patenterteilung: **20.08.2008**
(21) Anmeldenummer: 02722103.5
(22) Anmeldetag: 20.02.2002
(51) Int. Cl.: C07C 213/10, C07C 217/74

(54) **VERFAHREN ZUR ISOLIERUNG UND REINIGUNG VON (1RS,2RS)-2- (DIMETHYLAMINO)METHYL -1-(3-METHOXYPHENYL)-CYCLOHEXANOL**
METHOD FOR ISOLATING AND PURIFYING (1RS,2RS)-2- (DIMETHYLAMINO)METHYL -1-(3-METHOXYPHENYL)-CYCLOHEXANOL
PROCEDE POUR L'ISOLATION ET LA PURIFICATION DE (1RS,2RS)-2- (DIMETHYLAMINO)METHYL -1-(3-METHOXYPHENYL)-CYCLOHEXANOL

(30) Priorität: 21.02.2001 DE 10108308
(43) Veröffentlichungstag der Anmeldung: 26.11.2003
(73) Patentinhaber: Grünenthal GmbH, 52078 Aachen (DE)
(72) Erfinder: HELL, Wolfgang, 52066 Aachen (DE)
(74) Vertreter: Kutzenberger, Helga
(86) Internationale Anmeldenummer: PCT/EP2002/001764
(87) Internationale Veröffentlichungsnummer: WO 2002/066414

(56) Entgegenhaltungen:
- WO-A-00/78705
- DE-A- 19 940 740
- US-A- 5 877 351

## Beschreibung

Die vorliegende Erfindung betrifft ein Verfahren zur Isolierung und Reinigung von (1 RS,2RS)-2-[(Dimethylamino)methyl]-1-(3-methoxyphenyl)-cyclohexanol als Saccharinat aus einem Gemisch bestehend aus den Diastereomeren (1 RS,2RS)-2-[(Dimethylamino)methyl]-1-(3-methoxyphenyl)-cyclohexanol und (1 SR,2RS)-2-[(Dimethylamino)methyl]-1-(3-methoxyphenyl)-cyclohexanol sowie gegebenenfalls Verunreinigungen.

Der pharmazeutische Wirkstoff (1RS,2RS)-2-[(Dimethylamino)methyl]-1-(3-methoxyphenyl)-cyclohexanol, der auch unter der Bezeichnung Tramadol am Markt erhältlich ist, wird häufig in Form seines Hydrochlorids als Analgetikum eingesetzt. Üblicherweise wird dieser Wirkstoff u.a. über eine Grignardreaktion hergestellt, wobei das (1 RS,2RS)-Diastereomere im Gemisch mit dem entsprechenden (1SR,2RS)-Diastereomeren erhalten wird, von dem es vor der Formulierung zu einem Arzneimittel abgetrennt werden muß.

Zur Abtrennung des (1 RS,2RS)-Diastereomeren von dem entsprechenden (1SR,2RS)-Diastereomeren sind verschiedene Verfahren bekannt. Sie beruhen u.a. auf der Umsetzung des (1 RS,2RS)/(1 SR,2RS)-Diastereomerengemisches mit Mineralsäuren und einer anschließenden fraktionierten Kristallisation aus organischen Lösungsmitteln. Nachteilig ist bei diesen Verfahren, daß jeweils mehrere Fraktionen erhalten werden, die separat voneinander aufzuarbeiten sind, wodurch die Wirtschaftlichkeit dieser Verfahren gemindert wird. Ferner kann der Einsatz konzentrierter Mineralsäuren zum Auftreten unerwünschter Zersetzungsprodukte führen, die die Reinigung des (1 RS,2RS)-Diastereomeren erschweren und dessen Ausbeute vermindern. Desweiteren läßt sich mit den bekannten Verfahren eine Abtrennung des (1 RS,2RS)-Diastereomeren in der Regel nur erreichen, wenn der Anteil dieses Diastereomeren in dem zu trennenden Diastereomerengemisch ungefähr 75 Gew.-% oder mehr beträgt.

In der US 5,877,351 wird ein Verfahren zur Isolierung und Reinigung des (1RS,2RS)-Diastereomeren aus einem Reaktionsgemisch beschrieben, welches neben dem (1 RS,2RS)/(1SR,2RS)-Diastereomerengemisch auch Verunreinigungen aus der vorgängigen Grignardreaktion aufweist. Gemäß diesem Verfahren wird eine Abtrennung des (1RS,2RS)-Diastereomeren in Form des entsprechenden Hydrobromids durch Zugabe einer wäßrigen Lösung von Bromwasserstoff zu der Reaktionsmischung erreicht. Nachteilig ist bei diesem Verfahren, daß das erhaltene Hydrobromid vor der Formulierung zu dem Arzneimittel üblicherweise in das entsprechende Hydrochloridsalz übergeführt werden muß.

Eine Aufgabe der vorliegenden Erfindung war es daher, ein Verfahren zur Isolierung und Reinigung von (1 RS,2RS)-2-[(Dimethylamino)methyl]-1-(3-methoxyphenyl)-cyclohexanol zur Verfügung zu stellen, mit dem dieser Wirkstoff nicht nur in hoher Reinheit und in sehr guten Ausbeuten erhalten wird, sondern auch als eine Verbindung vorliegt, die direkt zur Herstellung eines Arzneimittels eingesetzt werden kann.

Erfindungsgemäß wird diese Aufgabe durch ein Verfahren zur Isolierung und Reinigung von (1 RS,2RS)-2-[(Dimethylamino)methyl]-1-(3-methoxyphenyl)-cyclohexanol als Saccharinat aus einem Gemisch bestehend aus den Diastereomeren (1 RS,2RS)-2-[(Dimethylamino)methyl]-1-(3-methoxyphenyl)-cyclohexanol und (1 SR,2RS)-2-[(Dimethylamino)methyl]-1-(3-methoxyphenyl)-cyclohexanol sowie gegebenenfalls Verunreinigungen gelöst, indem dieses Gemisch in einem flüssigen Reaktionsmedium mit einer Polarität von wenigstens 38 kcal/mol mit Saccharin umgesetzt wird, der so erhaltene kristalline Niederschlag des Saccharinats des (1RS,2RS)-2-[(Dimethylamino)methyl]-1-(3-methoxyphenyl)-cyclohexanol-Diastereomeren von der Mutterlauge abgetrennt, gegebenenfalls zumindest einmal gewaschen und/oder gegebenenfalls zumindest einmal umkristallisiert und anschließend getrocknet wird.

Unter Polarität im Sinne der vorliegenden Erfindung wird die empirisch bestimmte Lösungsmittelpolarität E_{T}(30) verstanden, die mit Hilfe des negativ solvatochromen Pyridinium-N-Phenolat Betainfarbstoffes der nachstehenden Formel I durch Messung der längstwelligen Absorptionsbande im sichtbaren/nahen Infrarot-(Vis/NIR)-Bereich bestimmt wird.

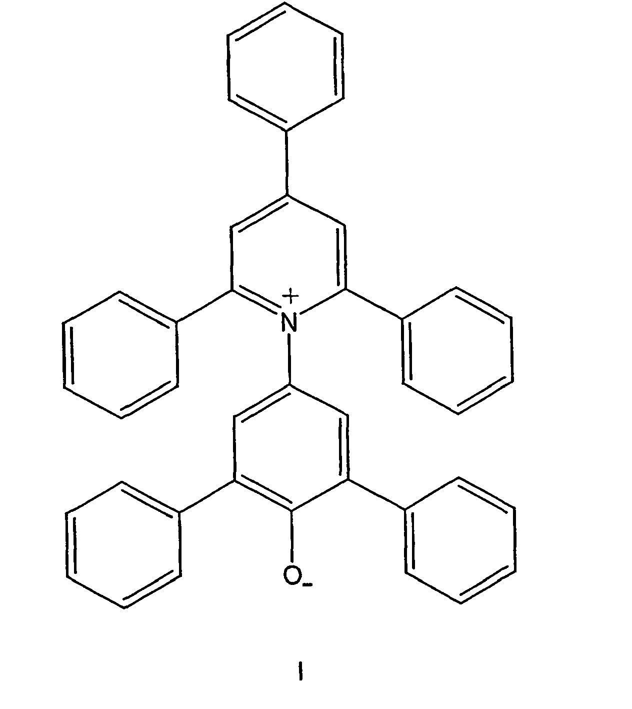

Die Methode zur Bestimmung dieser E_{T}(30)-Werte sowie die entsprechenden Werte für eine Vielzahl von Reaktionsmedien sind beispielsweise in C. Reichardt, Chem. Rev. 1994, 94, Seiten 2319-2358, C. Reichardt und G. Schäfer, Liebigs Ann., 1995, Seiten 1579-1582 sowie in R. Eberhardt et al, 1997, Liebigs Ann./Recueil, Seiten 1195-1199 beschrieben.

In einer bevorzugten Ausführungsform des erfindungsgemäßen Verfahrens wird ein Reaktionsmedium mit einer Polarität von wenigstens 45 kcal/mol, besonders bevorzugt wenigstens 55 kcal/mol verwendet.

Unter dem (1 RS,2RS)-2-[(Dimethylamino)methyl]-1-(3-methoxyphenyl)-cyclohexanol-Diasteromeren wird im Sinne der vorliegenden Erfindung das Racemat aus den Verbindungen der nachstehend abgebildeten Formeln IIa und IIb verstanden:

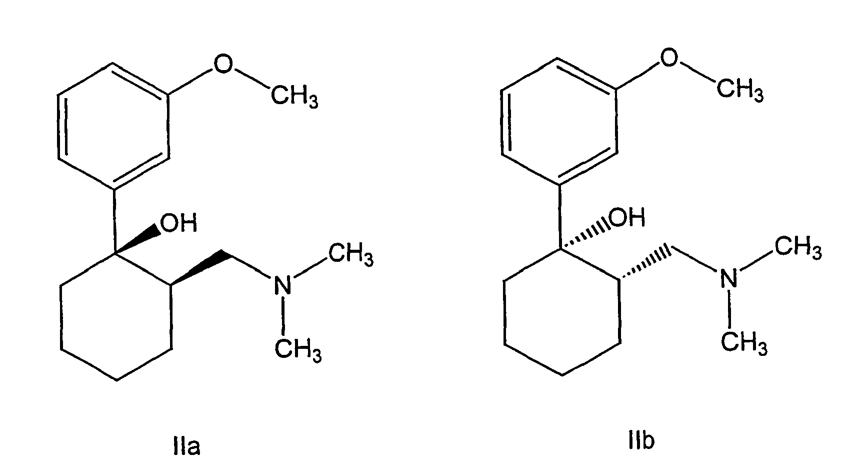

Das Enantiomer der Formel IIa ist das (1 R,2R)-2-[(Dimethylamino)methyl]-1-(3-methoxyphenyl)-cyclohexanol, das Enantiomer der Formel IIb ist das (1 S,2S)-2-[(Dimethylamino)methyl]-1-(3-methoxyphenyl)-cyclohexanol.

Unter dem (1 SR,2RS)-2-[(Dimethylamino)methyl]-1-(3-methoxyphenyl)-cyclohexanol-Diasteromeren wird im Sinne der vorliegenden Erfindung das Racemat aus den Verbindungen der nachstehend abgebildeten Formeln IIIa und IIIb verstanden:

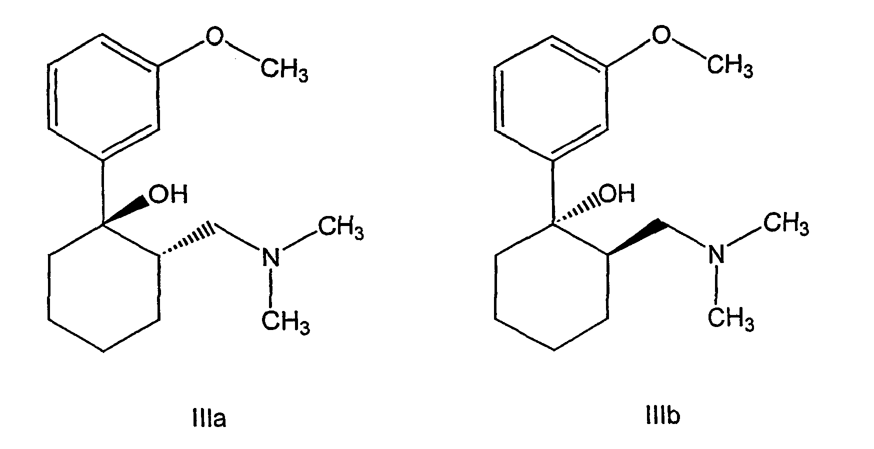

In einer bevorzugten Ausführungsform des erfindungsgemäßen Verfahrens weist das eingesetzte (1 RS,2RS)/(1 SR,2RS)-Diastereomerengemisch mindestens 50 Gew.-%, vorzugsweise mindestens 60 Gew.-% des (1RS,2RS)-2-[(Dimethylamino)methyl]-1-(3-methoxyphenyl)-cyclohexanol-Diastereomeren auf.

In einer bevorzugten Ausführungsform des erfindungsgemäßen Verfahrens wird als bei 20 °C und Normaldruck flüssiges Reaktionsmedium neben Wasser eine niedermolekulare organische Verbindung mit der angegebenen Polarität, wie ein aliphatischer Alkohol, vorzugsweise mit C₁-C₄, ein aliphatisches Keton, vorzugsweise mit C₃-C₇, ein aliphatischer Ester, vorzugsweise mit C₂-C₆, ein aliphatischer und/oder aromatischer Ester, vorzugsweise mit C₇-C₁₂, ein aliphatischer oder aromatischer Ether, vorzugsweise ein aliphatischer Ether mit C₄-C₆, ein Halogenalkan, vorzugsweise mit C₁-C₂, ein aliphatisches oder aromatisches Nitril, ein Polyol, vorzugsweise ein Polyol mit C₂-C₁₀ oder eine Mischung aus wenigstens zwei dieser vorstehend genannten Verbindungen eingesetzt. Besonders bevorzugt wird als flüssiges Reaktionsmedium in den erfindungsgemäßen Verfahren Wasser, Methanol, Ethanol, n-Propanol, Isopropanol, n-Butanol, iso-Butanol, Essigsäureethylester, Essigsäure-n-Butylester, Ameisensäuremethylester, Methylethylketon, Diisopropylether, Anisol, Ethylenglykol, Propylenglykol, Aceton oder eine Mischung aus wenigstens zwei dieser vorstehend Verbindungen eingesetzt. Ganz besonders bevorzugte Mischungen von Wasser und einer organischen Verbindung sind Mischungen aus Wasser und Ethanol oder aus Wasser und Aceton.

Sofern in dem erfindungsgemäßen Verfahren eine Mischung aus Wasser und einer der vorstehend genannten organischen Verbindungen zum Einsatz kommt, kann diese vorzugsweise 60 bis 95 Gew.-% der organischen Verbindung und 5 bis 40 Gew.-% Wasser, besonders bevorzugt 70 bis 90 Gew.-% der organischen Verbindung und 10 bis 30 Gew.-% Wasser, ganz besonders bevorzugt 75 bis 85 Gew.-% der organischen Verbindung und 15 bis 25 Gew.-% Wasser, jeweils bezogen auf die gesamte Menge des Reaktionsmediums, aufweisen.

In einer weiteren bevorzugten Ausführungsform des erfindungsgemäßen Verfahrens wird das Gemisch aus den (1 RS,2RS)/(1SR,2RS)-Diastereomeren und gegebenenfalls Verunreinigungen während und/oder unmittelbar nach der Umsetzung mit dem Saccharin gekühlt. Die für eine optimale Ausbeute des (1 RS,2RS)-Diastereomeren einzustellende Temperatur hängt beispielsweise von dem verwendeten Reaktionsmedium ab und kann vom Fachmann mit Hilfe von einfachen Vorversuchen ermittelt werden. Vorzugsweise wird auf eine Temperatur im Bereich von 2 bis 15 °C, besonders bevorzugt im Bereich von 5 bis 10 °C gekühlt, wobei das Reaktionsmedium bei diesen Temperaturen flüssig bleiben soll.

Ebenfalls bevorzugt wird das Reaktionsgemisch vor der Abtrennung des kristallinen Niederschlags gerührt. Die Zeit, die das Reaktionsgemisch gerührt werden muß, um eine optimale Ausbeute des gewünschten (1 RS,2RS)-Diasteromeren zu erhalten, hängt beispielsweise von dem verwendeten Reaktionsmedium bzw. von der Temperatur ab und kann vom Fachmann durch einfache Vorversuche bestimmt werden. Vorzugsweise wird das Reaktionsgemisch 5 bis 25 Stunden, besonders bevorzugt 10 bis 20 Stunden gerührt. Als Rührvorrichtungen kommen hierzu die üblichen, dem Fachmann bekannten Rührvorrichtungen in Frage, wie beispielsweise ein Ankerrührer.

Die Abtrennung des kristallinen Niederschlags von der Mutterlauge kann nach üblichen, dem Fachmann bekannten Methoden erfolgen. Vorzugsweise erfolgt die Abtrennung des kristallinen Niederschlags durch Zentrifugation, Saugfiltration, Dekantierung oder eine Kombination von wenigstens zwei dieser vorstehend genannten Methoden.

Gegebenfalls kann es vorteilhaft sein, den abgetrennten kristallinen Niederschlag einmal oder mehrfach zu waschen, um die Reinheit des (1 RS,2RS)-2-[(Dimethylamino)methyl]-1-(3-methoxyphenyl)-cyclohexanolsaccharinats weiter zu verbessern.
Vorzugsweise wird zum Waschen des kristallinen Niederschlags das Reaktionsmedium verwendet, in dem auch die Umsetzung mit dem Saccharin durchgeführt worden ist.

Vorzugsweise wird das zum Waschen des Niederschlags zum Einsatz kommende Reaktionsmedium gekühlt, um zu verhindern, daß der kristalline Niederschlag sich darin teilweise oder vollständig auflöst. Vorzugsweise wird das Reaktionsmedium auf eine Temperatur von 2 bis 15 °C, besonders bevorzugt von 5 bis 10 °C gekühlt.

Zur weiteren Verbesserung der Reinheit des (1 RS,2RS)-Diastereomeren kann es ferner vorteilhaft sein, den kristallinen Niederschlag des (1 RS,2RS)-2-[(Dimethylamino)methyl]-1-(3-methoxyphenyl)-cyclohexanolsaccharinats einmal oder mehrfach umzukristallisieren oder diesen in einem geeigneten Medium zu rühren. Diese Umkristallisierung kann nach üblichen, dem Fachmann bekannten Methoden erfolgen. Vorzugsweise wird für die Umkristallisierung des kristallinen Niederschlags das Reaktionsmedium verwendet, in dem die Umsetzung mit dem Saccharinat durchgeführt worden ist.

Die Trocknung des nach der Umsetzung mit Saccharin erhaltenen kristallinen Niederschlags bzw. des gewaschenen und/oder umkristallisierten Niederschlags kann nach üblichen, dem Fachmann bekannten Methoden erfolgen. Vorzugsweise erfolgt eine Trocknung des kristallinen Niederschlags bereits bei oder unmittelbar nach dem Abtrennen von der Mutterlauge durch Saugfiltration an der Luft und/oder Trocknen im Trockenschrank, ggf. unter Anlegen eines Vakuums. Sofern der Niederschlag im Trockenschrank getrocknet wird, beträgt die bevorzugte Temperatur 35 bis 45 °C.

In einer weiteren bevorzugten Ausführungsform des erfindungsgemäßen Verfahrens wird das zu trennende (1 RS,2RS)/(1 SR,2RS)-Diastereomerengemisch direkt nach der zur Herstellung des Gemisches durchgeführten Grignardreaktion, d.h. ohne jegliche Aufreinigung, mit dem Saccharin umgesetzt. Als Verunreinigungen liegen dann Nebenprodukte vor, die aus dieser Grignardreaktion stammen. Diese Grignardreaktion sowie dabei auftretende mögliche Nebenprodukte werden beispielsweise in der US 5,877,351 beschrieben. Die entsprechende Beschreibung daraus wird hiermit als Referenz eingeführt und gilt somit als Teil der Offenbarung.

Das zu trennende (1RS,2RS)/(1SR,2RS)-Diastereomerengemisch kann aber auch vor der Umsetzung mit dem Saccharin von Verunreinigungen befreit werden, beispielsweise durch Destillation bei verminderten Druck, wie beispielsweise in der US 5,877,351 oder US 3,652,589 beschrieben.

Die Grignardreaktion zur Herstellung des Diastereomerengemisches kann auch in Gegenwart eines Additivs, beispielsweise in Gegenwart eines Amins oder eines Ethers, durchgeführt werden, um ein verbessertes (1 RS,2RS)-/(1 SR,2RS)-Diastereomerenverhältnis zu erzielen, wie beispielsweise in der WO 99/61405 beschrieben.

Das nach den erfindungsgemäßen Verfahren erhaltene (1RS,2RS)-2-[(Dimethylamino)methyl]-1-(3-methoxyphenyl)-cyclohexanolsaccharinat eignet sich direkt zur Formulierung eines Arzneimittels. Der Wirkstoff (1 RS,2RS)-2-[(Dimethylamino)methyl]-1-(3-methoxyphenyl)-cyclohexanol kann aber, falls erforderlich, aus dem Saccharinat auch als freie Base erhalten werden.

In einer weiteren bevorzugten Ausführungsform des erfindungsgemäßen Verfahrens wird daher das (1 RS,2RS)-2-[(Dimethylamino)methyl]-1-(3-methoxyphenyl)-cyclohexanolsaccharinat durch Umsetzung mit einer geeigneten Base, wie z.B. Natriumhydroxid, in einem geeigneten organischen Lösungsmittel oder Lösungsmittelgemisch, wie beispielsweise Tetrahydrofuran oder Toluol freigesetzt. Hierzu wird die Base äquimolar oder im Überschuß, bezogen auf das (1 RS,2RS)-2-[(Dimethylamino)methyl]-1-(3-methoxyphenyl)-cyclohexanolsaccharinat, eingesetzt. Die so erhaltene freie Base des (1 RS,2RS)-Diastereomeren kann dann nach üblichen, dem Fachmann bekannten Methoden gereinigt und isoliert werden.

Die freie Base des (1 RS,2RS)-Diastereomeren kann durch die Umsetzung mit entsprechenden Säuren nach üblichen, dem Fachmann bekannten Methoden in die entsprechenden Wirkstoffsalze überführt werden. Die Überführung des (1 RS,2RS)-2-[(Dimethylamino)methyl]-1-(3-methoxyphenyl)-cyclohexanols in das entsprechende Hydrochlorid-Salz kann beispielsweise durch Umsetzung mit einer wäßrigen Lösung von Chlorwasserstoff erfolgen.

Das erfindungsgemäße Verfahren hat den Vorteil, daß der Wirkstoff (1 RS,2RS)-2-[(Dimethylamino)methyl]-1-(3-methoxyphenyl)-cyclohexanol in sehr guten Ausbeuten und mit einer sehr hohen Reinheit erhalten wird. Vorteilhaft ist weiterhin, daß der Wirkstoff nach der Isolierung und Reinigung in Form des entsprechenden Saccharinats vorliegt, welches sich direkt zur Formulierung eines Arzneimittels eignet und nicht durch weitere Verfahrensschritte in ein anderes physiologisch verträgliches Salz, wie beispielsweise das entsprechende Hydrochlorid, übergeführt werden muß.

Die Reinheit des (1 RS,2RS)-Diasteromeren bzw. eines entsprechenden Salzes, d.h. das Verhältnis der (1RS,2RS)/(1SR,2RS)-Diasteromeren in dem nach den erfindungsgemäßen Verfahren erhaltenen Produkt, kann nach üblichen, dem Fachmann bekannten Methoden bestimmt werden. Vorzugsweise wird das Verhältnis der Diastereomeren mittels HPLC auf einer V2A-Stahl-Säule (Länge 12,5 cm, Durchmesser 3,0 mm) und einer Nucleosil 100-5µ C8 HD Trennphase gegen einen geeigneten Standard bestimmt, wobei die Durchflußmenge auf 0,7 ml/min, die Temperatur auf 25 °C eingestellt wird. Die Detektion erfolgt bei einer Wellenlänge von 270 nm.

Im folgenden wird die Erfindung anhand von Beispielen erläutert. Diese Erläuterungen sind lediglich beispielhaft.

### Beispiele:

In den erfindungsgemäßen Beispielen 1 bis 5 wird ein Gemisch bestehend aus (1 RS,2RS)-2-[(Dimethylamino)methyl]-1-(3-methoxyphenyl)-cyclohexanol und (1SR,2RS)-2-[(Dimethylamino)methyl]-1-(3-methoxyphenyl)-cyclohexanol und weiteren Verunreinigungen erhalten aus der Grignardreaktion gemäß US 3,652,589 eingesetzt. Das so erhaltene Gemisch aus den Diasteromeren und den Verunreinigungen wurde in den Beispielen 1 bis 5 direkt nach der Grignardreaktion, d.h. ohne jede weitere Aufreinigung, eingesetzt.

### Beispiel 1:

In einer 10 Liter Doppelmantelreaktionsanlage mit elektrischem Ankerrührer, Rückflußkühler, Thermometer und Kühl-/Heizaggregat (Firma Huber, Unistat 161 W) wurden 1,5 kg des nach der oben angegebenen Grignardreaktion erhaltenen Gemisches in 5,0 Litern Ethanol mit einer Polarität von 51,9 kcal/mol bei einer Temperatur von 20 °C gelöst. Zu dieser Lösung wurden 1,04 kg Saccharin gegeben. Anschließend wurde dieses Reaktionsgemisch auf eine Temperatur von 8 °C abgekühlt und 16 Stunden bei dieser Temperatur nachgerührt. Hierbei bildete sich ein kristalliner Niederschlag von (1RS,2RS)-2-[(Dimethylamino)methyl]-1-(3-methoxyphenyl)-cyclohexanolsaccharinat, der durch Saugfiltration im Vakuum mit einer Fritte der Größe G3 von der Mutterlauge abgetrennt wurde. Anschließend wurde der Niederschlag zweimal mit jeweils 2,0 Litern Ethanol gewaschen, das zuvor auf eine Temperatur von 8 °C abgekühlt wurde, und dann im Vakuumtrockenschrank bei einer Temperatur von 40 °C und einem Druck von 20 mbar für 16 Stunden getrocknet. Die Ausbeute des so erhaltenen Produktes betrug 1,90 kg (entsprechend 75 % des theoretisch berechneten Wertes) mit einem Gehalt an (1 RS,2RS)-2-[(Dimethylamino)methyl]-1-(3-methoxyphenyl)-cyclohexanolsaccharinat von > 95 Gew.-%.

### Beispiel 2:

In einer 20 Liter Doppelmantelreaktionsanlage mit elektrischem Ankerrührer, Rückflußkühler, Thermometer und Kühl-/Heizaggregat (Firma Huber, Unistat 161 W) wurden 1,5 kg des nach der oben angegebenen Grignardreaktion erhaltenen Gemisches in 12,5 Litern Essigsäureethylester mit einer Polarität von 38,1 kcal/mol bei einer Temperatur von 20 °C gelöst. Zu dieser Lösung wurden 1,04 kg Saccharin gegeben. Anschließend wurde dieses Reaktionsgemisch auf eine Temperatur von 8 °C abgekühlt und 16 Stunden bei dieser Temperatur nachgerührt. Hierbei bildete sich ein kristalliner Niederschlag von (1 RS,2RS)-2-[(Dimethylamino)methyl]-1-(3-methoxypheny')-cyclohexanolsaccharinat, der durch Saugfiltration im Vakuum auf einer Fritte der Größe G3 von der Mutterlauge abgetrennt wurde. Der kristalline Niederschlag wurde zweimal mit jeweils 2,0 Litern Essigsäureethylester gewaschen, welcher zuvor auf eine Temperatur von 8 °C abgekühlt wurde, und anschließend im Vakuumtrockenschrank bei einer Temperatur von 40 °C und einem Druck von 20 mbar für 16 Stunden getrocknet. Die Ausbeute des so erhaltenen Produktes betrug 2,16 kg (entsprechend 85 % des theoretisch berechneten Wertes) mit einem Gehalt an (1RS,2RS)-2-[(Dimethylamino)methyl]-1-(3-methoxyphenyl)-cyclohexanolsaccharinat von > 92 Gew.-%.

### Beispiel 3:

In einer 10 Liter Doppelmantelreaktionsanlage mit elektrischem Ankerrührer, Rückflußkühler, Thermometer und Kühl-/Heizaggregat (Firma Huber, Unistat 161 W) wurden 1,5 kg des nach der oben angegebenen Grignardreaktion erhaltenen Gemisches in 4,0 Litern Ethanol und 1,0 Litern Wasser mit einer Polarität von 53,7 kcal/mol bei einer Temperatur von 20 °C gelöst. Zu dieser Lösung wurden 1,04 kg Saccharin gegeben. Anschließend wurde dieses Reaktionsgemisch auf eine Temperatur von 8 °C abgekühlt und 16 Stunden bei dieser Temperatur nachgerührt. Hierbei bildete sich ein kristalliner Niederschlag von (1 RS,2RS)-2-[(Dimethylamino)methyl)-1-(3-methoxyphenyl)-cyclohexanolsaccharinat, der durch Saugfiltration im Vakuum über eine Fritte der Größe G3 von der Mutterlauge abgetrennt wurde. Der kristalline Niederschlag wurde zweimal mit jeweils 2,0 Litern Ethanol gewaschen, welches zuvor auf eine Temperatur von 8 °C abgekühlt wurde, und anschließend im Vakuumtrockenschrank bei einer Temperatur von 40 °C und einem Druck von 20 mbar 16 Stunden getrocknet. Die Ausbeute des so erhaltenen Produktes betrug 1,53 kg (entsprechend 60 % des theoretisch berechneten Wertes) mit einem Gehalt an (1 RS,2RS)-2-[(Dimethylamino)methyl]-1-(3-methoxyphenyl)-cyclohexanolsaccharinat von > 99 Gew.-%.

### Beispiel 4:

In einer 10 Liter Doppelmantelreaktionsanlage mit elektrischem Ankerrührer, Rückflußkühler, Thermometer und Kühl-/Heizaggregat (Firma Huber, Unistat 161 W) wurden 1,5 kg des nach der oben angegebenen Grignardreaktion erhaltenen Gemisches in 5,0 Litern Wasser mit einer Polarität von 63,1 kcal/mol bei einer Temperatur von 20 °C gelöst. Zu dieser Lösung wurden 1,04 kg Saccharin gegeben. Anschließend wurde dieses Reaktionsgemisch auf eine Temperatur von 8 °C abgekühlt und 16 Stunden bei dieser Temperatur nachgerührt. Hierbei bildete sich ein kristalliner Niederschlag von (1 RS,2RS)-2-[(Dimethylamino)methyl]-1-(3-methoxyphenyl)-cyclohexanolsaccharinat, der durch Saugfiltration im Vakuum über eine Fritte der Größe G3 von der Mutterlauge abgetrennt wurde. Der kristalline Niederschlag wurde zweimal mit jeweils 2,0 Litern Ethanol gewaschen, welches zuvor auf eine Temperatur von 8 °C abgekühlt wurde, und anschließend im Vakuumtrockenschrank bei einer Temperatur von 40 °C und einem Druck von 20 mbar für 16 Stunden getrocknet. Die Ausbeute des so erhaltenen Produktes betrug 2,16 kg (entsprechend 85 % des theoretisch berechneten Wertes) mit einem Gehalt an (1 RS,2RS)-2-[(Dimethylamino)methyl]-1-(3-methöxyphenyl)-cyclohexanolsaccharinat von > 90 Gew.-%.

### Beispiel 5:

In einer 20 Liter Doppelmantelreaktionsanlage mit elektrischem Ankerrührer, Rückflußkühler, Thermometer und Kühl/Heizgerät (Firma Huber, Unistat 161 W) wurden 1,0 kg des nach der oben angegebenen Grignardreaktion erhaltenen Gemisches in 14 Litern Aceton mit einer Polarität von 42,2 kcal/mol bei einer Temperatur von 20 °C gelöst. Zu dieser Lösung wurden 0,69 kg Saccharin gegeben. Anschließend wurde dieses Reaktionsgemisch auf eine Temperatur von 8 °C abgekühlt und 16 Stunden bei dieser Temperatur nachgerührt. Hierbei bildete sich ein kristalliner Niederschlag von (1 RS,2RS)-2-[(Dimethylamino)methyl]-1-(3-methoxyphenyl)-cyclohexanolsaccharinat, der durch Saugfiltration im Vakuum mit einer Fritte der Größe G3 von der Mutterlauge abgetrennt wurde. Der kristalline Niederschlag wurde zweimal mit jeweils 3 Litern Aceton gewaschen, welches zuvor auf eine Temperatur von 8 °C abgekühlt wurde, und anschließend im Vakuumschrank bei einer Temperatur von 40 °C und einem Druck von 20 mbar für 16 Stunden getrocknet. Die Ausbeute des so erhaltenen Produktes betrug 1,09 kg (entsprechend 64 % des theoretisch berechneten Wertes) mit einem Gehalt an (1 RS,2RS)-2-[(Dimethylamino)methyl]-1-(3-methoxyphenyl)-cyclohexanolsaccharinat von > 96 Gew.-%.

## Patentansprüche

1. Verfahren zur Isolierung und Reinigung von (1 RS,2RS)-2-[(Dimethylamino)methyl]-1-(3-methoxyphenyl)-cyclohexanol als Saccharinat aus einem Gemisch bestehend aus den Diastereomeren (1 RS,2RS)-2-[(Dimethylamino)methyl]-1-(3-methoxyphenyl)-cyclohexanol und (1SR,2RS)-2-[(Dimethylamino)methyl]-1-(3-methoxyphenyl)-cyclohexanol sowie gegebenenfalls Verunreinigungen, **dadurch gekennzeichnet, daß** das Gemisch in einem bei 20 °C und Normaldruck flüssigen Reaktionsmedium mit einer Polarität von mindestens 38 kcal/mol mit Saccharin umgesetzt und der so erhaltene kristalline Niederschlag des Saccharinats des (1RS,2RS)-2-[(Dimethylamino)methyl]-1-(3-methoxyphenyl)-cyclohexanol-Diastereomeren von der Mutterlauge abgetrennt wird.

2. Verfahren gemäß Anspruch 1, **dadurch gekennzeichnet, daß** der kristalline Niederschlag zumindest einmal gewaschen und/oder zumindest einmal umkristallisiert und anschließend getrocknet wird.

3. Verfahren gemäß Anspruch 1 oder 2, **dadurch gekennzeichnet, daß** die Polarität des Reaktionsmediums wenigstens 45 kcal/mol, vorzugsweise wenigstens 55 kcal/mol beträgt.

4. Verfahren gemäß einem der Ansprüche 1 bis 3, **dadurch gekennzeichnet, daß** das Gemisch mindestens 50 Gew.-%, vorzugsweise mindestens 60 Gew.-% des (1 RS,2RS)-2-[(Dimethylamino)methyl]-1-(3-methoxyphenyl)-cyclohexanol-Diastereomeren aufweist.

5. Verfahren gemäß einem der Ansprüche 1 bis 4, **dadurch gekennzeichnet, daß** als Reaktionsmedium Wasser, ein aliphatischer Alkohol, ein aliphatisches Keton, ein aliphatischer Ester, ein aliphatischer und/oder aromatischer Ester, ein aliphatischer und/oder aromatischer Ether, ein Polyol, ein Halogenalkan, ein aliphatisches oder aromatisches Nitril oder eine Mischung aus wenigstens zwei dieser vorstehend genannten Verbindungen eingesetzt wird.

6. Verfahren gemäß Anspruch 5, **dadurch gekennzeichnet, daß** als Reaktionsmedium Wasser, ein C₁₋₄-Alkohol, ein aliphatisches C₃₋₇-Keton, ein aliphatischer C₂₋₆-Ester, ein aliphatischer und/oder aromatischer C₇₋₁₂-Ester, ein aliphatischer C₄₋₆-Ether, ein C₁₋₂-Halogenalkan, ein aliphatisches oder aromatisches Nitril, ein C₂₋₁₀-Polyol oder eine Mischung aus wenigstens zwei dieser vorstehend genannten Verbindungen eingesetzt wird.

7. Verfahren gemäß Anspruch 5 oder 6, **dadurch gekennzeichnet, daß** als Reaktionsmedium Wasser, Methanol, Ethanol, n-Propanol, Isopropanol, n-Butanol, iso-Butanol, Essigsäureethylester, Essigsäure-n-Butylester, Ameisensäuremethylester, Methylethylketon, Diisopropylether, Anisol, Ethylenglykol, Propylenglykol, Aceton oder eine Mischung aus wenigstens zwei dieser vorstehend genannten Verbindungen eingesetzt wird.

8. Verfahren gemäß 7, **dadurch gekennzeichnet, daß** als Reaktionsmedium eine Mischung aus Wasser und Ethanol oder aus Wasser und Aceton eingesetzt wird.

9. Verfahren gemäß einem der Ansprüche 5 bis 8, **dadurch gekennzeichnet, daß** eine Mischung aus 60 bis 95 Gew.-% einer organischen Verbindung und 5 bis 40 Gew.-% Wasser, vorzugsweise 70 bis 90 Gew.-% einer organischen Verbindung und 10 bis 30 Gew.-% Wasser, besonders bevorzugt 75 bis 85 Gew.-% einer organischen Verbindung und 15 bis 25 Gew.-% Wasser, jeweils bezogen auf die gesamte Menge des Reaktionsmediums, vorliegt.

10. Verfahren gemäß einem der Ansprüche 1 bis 9, **dadurch gekennzeichnet, daß** während und/oder unmittelbar nach der Umsetzung mit dem Saccharin gekühlt wird.

11. Verfahren gemäß Anspruch 10, **dadurch gekennzeichnet, daß** auf eine Temperatur von 2 bis 15 °C, bevorzugt von 5 bis 10 °C gekühlt wird.

12. Verfahren gemäß einem der Ansprüche 1 bis 11, **dadurch gekennzeichnet, daß** vor der Abtrennung des kristallinen Niederschlags gerührt wird.

13. Verfahren gemäß Anspruch 12, **dadurch gekennzeichnet, daß** 5 bis 25 Stunden, vorzugsweise 10 bis 20 Stunden gerührt wird.

14. Verfahren gemäß einem der Ansprüche 1 bis 13, **dadurch gekennzeichnet, daß** der Niederschlag durch Zentrifugation, Saugfiltration, Dekantierung oder eine Kombination dieser Methoden von der Mutterlauge abgetrennt wird.

15. Verfahren gemäß einem der Ansprüche 2 bis 14, **dadurch gekennzeichnet, daß** das Waschen des Niederschlags mit dem Reaktionsmedium erfolgt.

16. Verfahren gemäß Anspruch 15, **dadurch gekennzeichnet, daß** das Reaktionsmedium gekühlt wird.

17. Verfahren gemäß Anspruch 16, **dadurch gekennzeichnet, daß** das Reaktionsmedium auf eine Temperatur von 2 bis 15 °C, vorzugsweise von 5 bis 10 °C gekühlt wird.

18. Verfahren gemäß einem der Ansprüche 1 bis 17, **dadurch gekennzeichnet, daß** als Verunreinigungen Nebenproduke vorliegen, die aus der Grignardreaktion zur Herstellung des Diastereomerengemisches von (1 RS,2RS)-2-[(Dimethylamino)methyl]-1-(3-methoxyphenyl)-cyclohexanol und (1SR,2RS)-2-[(Dimethylamino)methyl]-1-(3-methoxyphenyl)-cyclohexanol stammen.

19. Verfahren gemäß einem der Ansprüche 1 bis 18, **dadurch gekennzeichnet, daß** das (1 RS,2RS)-2-[(Dimethylamino)methyl]-1-(3-methoxyphenyl)-cyclohexanolsaccharinat in einem Lösungsmittel oder Lösungsmittelgemisch mit wenigstens einer Base freigesetzt und die so erhaltene freie Base (1 RS,2RS)-2-[(Dimethylamino)methyl]-1-(3-methoxyphenyl)-cyclohexanol nach üblichen Methoden gereinigt und isoliert wird.

20. Verfahren gemäß Anspruch 19, **dadurch gekennzeichnet, daß** die Base äquimolar oder im Überschuß, bezogen auf das (1 RS,2RS)-2-[(Dimethylamino)methyl]-1-(3-methoxyphenyl)-cyclohexanolsaccharinat, eingesetzt wird.

21. Verfahren gemäß einem der Ansprüche 19 oder 20, **dadurch gekennzeichnet, daß** das (1 RS,2RS)-2-[(Dimethylamino)methyl]-1-(3-methoxyphenyl)-cyclohexanol durch Umsetzung mit einer Säure in das entsprechende Wirkstoffsalz übergeführt wird.

## Claims

1. Process for isolating and purifying (1RS,2RS)-2-[(dimethylamino)methyl]-1-(3-methoxyphenyl)cyclohexanol as the saccharinate from a mixture consisting of the diastereomers (1RS,2RS)-2-[(dimethylamino)methyl]-1-(3-methoxyphenyl)cyclohexanol and (1SR,2RS)-2-[(dimethylamino)methyl]-1-(3-methoxyphenyl)cyclohexanol and also in some cases impurities, **characterized in that** the mixture is reacted with saccharin in a reaction medium which is liquid at 20°C and atmospheric pressure and has a polarity of at least 38 kcal/mol, and the thus-obtained crystalline precipitate of the saccharinate of (1RS,2RS)-2-[(dimethylamino)methyl]-1-(3-methoxyphenyl)cyclohexanol diastereomers is removed from the mother liquor.

2. Process according to Claim 1, **characterized in that** the crystalline precipitate is washed at least once and/or recrystallized and subsequently dried at least once.

3. Process according to Claim 1 or 2, **characterized in that** the polarity of the reaction medium is at least 45 kcal/mol, preferably at least 55 kcal/mol.

4. Process according to one of Claims 1 to 3, **characterized in that** the mixture comprises at least 50% by weight, preferably at least 60% by weight, of the (1RS,2RS)-2-[(dimethylamino)methyl]-1-(3-methoxyphenyl)cyclohexanol diastereomer.

5. Process according to one of Claims 1 to 4, **characterized in that** the reaction medium used is water, an aliphatic alcohol, an aliphatic ketone, an aliphatic ester, an aliphatic and/or aromatic ester, an aliphatic and/or aromatic ether, a polyol, a haloalkane, an aliphatic or aromatic nitrile or a mixture of at least two of these aforementioned compounds.

6. Process according to Claim 5, **characterized in that** the reaction medium used is water, a C₁₋₄-alcohol, an aliphatic C₃₋₇-ketone, an aliphatic C₂₋₆-ester, an aliphatic and/or aromatic C₇₋₁₂-ester, an aliphatic C₄₋₆-ether, a C₁₋₂-haloalkane, an aliphatic or aromatic nitrile, a C₂₋₁₀-polyol or a mixture of at least two of these aforementioned compounds.

7. Process according to Claim 5 or 6, **characterized in that** the reaction medium used is water, methanol, ethanol, n-propanol, isopropanol, n-butanol, isobutanol, ethyl acetate, n-butyl acetate, methyl formate, methyl ethyl ketone, diisopropyl ether, anisole, ethylene glycol, propylene glycol, acetone or a mixture of at least two of these aforementioned compounds.

8. Process according to Claim 7, **characterized in that** the reaction medium used is a mixture of water and ethanol or of water and acetone.

9. Process according to one of Claims 5 to 8, **characterized in that** the mixture is of from 60 to 95% by weight of an organic compound and from 5 to 40% by weight of water, preferably from 70 to 90% by weight of an organic compound and from 10 to 30% by weight of water, more preferably from 75 to 85% by weight of an organic compound and from 15 to 25% by weight of water, based in each case on the entire amount of the reaction medium.

10. Process according to one of Claims 1 to 9, **characterized in that** the mixture is cooled during and/or immediately after the reaction with saccharin.

11. Process according to Claim 10, **characterized in that** the mixture is cooled to a temperature of from 2 to 15°C, preferably from 5 to 10°C.

12. Process according to one of Claims 1 to 11, **characterized in that** the mixture is stirred before the crystalline precipitate is removed.

13. Process according to Claim 12, **characterized in that** the mixture is stirred for from 5 to 25 hours, preferably for from 10 to 20 hours.

14. Process according to one of Claims 1 to 13, **characterized in that** the precipitate is removed from the mother liquor by centrifugation, suction filtration, decanting or a combination of these methods.

15. Process according to one of Claims 2 to 14, **characterized in that** the reaction medium is used to wash the precipitate.

16. Process according to Claim 15, **characterized in that** the reaction medium is cooled.

17. Process according to Claim 16, **characterized in that** the reaction medium is cooled to a temperature of from 2 to 15°C, preferably from 5 to 10°C.

18. Process according to one of Claims 1 to 17, **characterized in that** the impurities are byproducts which stem from the Grignard reaction for preparing the diastereomer mixture of (1RS,2RS)-2-[(dimethylamino)methyl]-1-(3-methoxyphenyl)cyclohexanol and (1SR,2RS)-2-[(dimethylamino)methyl]-1-(3-methoxyphenyl)cyclohexanol.

19. Process according to one of Claims 1 to 18, **characterized in that** the (1RS,2RS)-2-[(dimethyl-amino)methyl]-1-(3-methoxyphenyl)cyclohexanol saccharinate is released with at least one base in a solvent or solvent mixture and the thus-obtained free (1RS,2RS)-2-[(dimethylamino)methyl]-1-(3-methoxyphenyl)cyclohexanol base is purified and isolated by customary methods.

20. Process according to Claim 19, **characterized in that** the base is used in an equimolar amount or in excess, based on the (1RS,2RS)-2-[(dimethylamino)methyl]-1-(3-methoxyphenyl)cyclohexanol saccharinate.

21. Process according to one of Claims 19 or 20, **characterized in that** the (1RS,2RS)-2-[(dimethyl-amino)methyl]-1-(3-methoxyphenyl)cyclohexanol is converted to the corresponding active ingredient salt by reacting with an acid.

## Revendications

1. Procédé pour l'isolement et la purification de (1RS,2RS)-2-[(diméthylamino)méthyl]-1-(3-méthoxyphényl)-cyclohexanol sous forme de saccharinate à partir d'un mélange constitué par les diastéréoisomères (1RS,2RS)-2-[(diméthylamino)méthyl]-1-(3-méthoxyphényl)-cyclohexanol et (1SR,2RS)-2-[(diméthylamino)méthyl]-1-(3-méthoxyphényl)-cyclohexanol ainsi que le cas échéant des impuretés, **caractérisé en ce qu'**on transforme le mélange dans un mélange réactionnel liquide à 20°C et à la pression normale, présentant une polarité d'au moins 38 kcal/mole, avec de la saccharine et on sépare le précipité cristallin du saccharinate du diastéréoisomère (1RS,2RS)-2-[(diméthylamino)méthyl]-1-(3-méthoxyphényl)-cyclohexanol ainsi obtenu de la lessive-mère.

2. Procédé selon la revendication 1, **caractérisé en ce que** le précipité cristallin est lavé au moins une fois et/ou recristallisé au moins une fois et ensuite séché.

3. Procédé selon la revendication 1 ou 2, **caractérisé en ce que** la polarité du mélange réactionnel est d'au moins 45 kcal/mole, de préférence d'au moins 55 kcal/mole.

4. Procédé selon l'une quelconque des revendications 1 à 3, **caractérisé en ce que** le mélange présente au moins 50% en poids, de préférence au moins 60% en poids du diastéréoisomère (1RS,2RS)-2-[(diméthylamino)méthyl]-1-(3-méthoxyphényl)-cyclohexanol.

5. Procédé selon l'une quelconque des revendications 1 à 4, **caractérisé en ce qu'**on utilise comme mélange réactionnel de l'eau, un alcool aliphatique, une cétone aliphatique, un ester aliphatique, un ester aliphatique et/ou aromatique, un éther aliphatique et/ou aromatique, un polyol, un halogénoalcane, un nitrile aliphatique ou aromatique ou un mélange d'au moins deux de ces composés susmentionnés.

6. Procédé selon la revendication 5, **caractérisé en ce qu'**on utilise comme mélange réactionnel de l'eau, un alcool en C₁₋₄, une cétone aliphatique en C₃₋₇, un ester aliphatique en C₂₋₆, un ester aliphatique et/aromatique en C₇₋₁₂, un éther aliphatique en C₄₋₆, un halogénoalcane en C₁₋₂, un nitrile aliphatique ou aromatique, un polyol en C₂₋₁₀ ou un mélange d'au moins deux de ces composés susmentionnés.

7. Procédé selon la revendication 5 ou 6, **caractérisé en ce qu'**on utilise comme mélange réactionnel de l'eau, du méthanol, de l'éthanol, du n-propanol, de l'isopropanol, du n-butanol, de l'iso-butanol, de l'ester éthylique de l'acide acétique, de l'ester n-butylique de l'acide acétique, de l'ester méthylique de l'acide formique, de la méthyléthylcétone, du diisopropyléther, de l'anisol, de l'éthylèneglycol, du propylèneglycol, de l'acétone ou un mélange d'au moins deux de ces composés susmentionnés.

8. Procédé selon la revendication 7, **caractérisé en ce qu'**on utilise, comme mélange réactionnel, un mélange d'eau et d'éthanol ou d'eau et d'acétone.

9. Procédé selon l'une quelconque des revendications 5 à 8, **caractérisé en ce qu'**on est en présence d'un mélange de 60 à 95% en poids d'un composé organique et de 5 à 40% en poids d'eau, de préférence de 70 à 90% en poids d'un composé organique et de 10 à 30% en poids d'eau, de manière particulièrement préférée de 75 à 85% en poids d'un composé organique et de 15 à 25% en poids d'eau, à chaque fois par rapport à la quantité totale du mélange réactionnel.

10. Procédé selon l'une quelconque des revendications 1 à 9, **caractérisé en ce qu'**on refroidit pendant et/ou immédiatement après la transformation avec la saccharine.

11. Procédé selon la revendication 10, **caractérisé en ce qu'**on refroidit à une température de 2 à 15°C, de préférence de 5 à 10°C.

12. Procédé selon l'une quelconque des revendications 1 à 11, **caractérisé en ce qu'**on agite avant la séparation du précipité cristallin.

13. Procédé selon la revendication 12, **caractérisé en ce qu'**on agite pendant 5 à 25 heures, de préférence pendant 10 à 20 heures.

14. Procédé selon l'une quelconque des revendications 1 à 13, **caractérisé en ce que** le précipité est séparé par centrifugation, filtration par aspiration, décantation ou une combinaison de ces procédés de la lessive-mère.

15. Procédé selon l'une quelconque des revendications 2 à 14, **caractérisé en ce que** le lavage du précipité est réalisé avec le mélange réactionnel.

16. Procédé selon la revendication 15, **caractérisé en ce que** le mélange réactionnel est refroidi.

17. Procédé selon la revendication 16, **caractérisé en ce que** le mélange réactionnel est refroidi à une température de 2 à 15°C, de préférence de 5 à 10°C.

18. Procédé selon l'une quelconque des revendications 1 à 17, **caractérisé en ce que** les impuretés sont des produits secondaires qui proviennent de la réaction de Grignard pour la préparation du mélange des diastéréoisomères (1RS,2RS)-2-[(diméthylamino)méthyl]-1-(3-méthoxyphényl)-cyclohexanol et (1SR,2RS)-2-[(diméthylamino)méthyl]-1-(3-méthoxyphényl)-cyclohexanol.

19. Procédé selon l'une quelconque des revendications 1 à 18, **caractérisé en ce que** le saccharinate de (1RS,2RS)-2-[(diméthylamino)méthyl]-1-(3-méthoxyphényl)-cyclohexanol est libéré dans un solvant ou un mélange de solvants avec au moins une base et la base libre ainsi obtenue, le (1RS,2RS)-2-[(diméthylamino)méthyl]-1-(3-méthoxyphényl)-cyclohexanol, est purifiée et isolée selon les procédés usuels.

20. Procédé selon la revendication 19, **caractérisé en ce que** la base est utilisée de manière équimolaire ou en excès, par rapport au saccharinate de (1RS,2RS)-2-[(diméthylamino)méthyl]-1-(3-méthoxyphényl)-cyclohexanol.

21. Procédé selon l'une quelconque des revendications 19 ou 20, **caractérisé en ce que** le (1RS,2RS)-2-[(diméthylamino)méthyl]-1-(3-méthoxyphényl)-cyclohexanol est transformé par transformation avec un acide en sel de substance active correspondant.
